# EUROPÄISCHE PATENTSCHRIFT

(11) **EP 1 150 759 B1**
(45) Veröffentlichungstag und Bekanntmachung des Hinweises auf die Patenterteilung: **17.09.2003**
(21) Anmeldenummer: 00910598.2
(22) Anmeldetag: 21.01.2000
(51) Int. Cl.: B01D 9/00, C07C 51/43

(54) **VERFAHREN UND VORRICHTUNG ZUR REINIGUNG VON STOFFEN MITTELS KRISTALLISATION**
METHOD AND DEVICE FOR PURIFYING SUBSTANCES BY MEANS OF CRYSTALLISATION
PROCEDE ET DISPOSITIF POUR PURIFIER DES SUBSTANCES PAR CRISTALLISATION

(30) Priorität: 05.02.1999 DE 19904820
(43) Veröffentlichungstag der Anmeldung: 07.11.2001
(73) Patentinhaber: STOCKHAUSEN GmbH & Co. KG, 47805 Krefeld (DE)
(72) Erfinder: BUB, Günther, D-45772 Marl (DE)
(74) Vertreter: Kahlhöfer, Hermann, Dipl.-Phys.
(86) Internationale Anmeldenummer: EP0000441
(87) Internationale Veröffentlichungsnummer: WO00045928

(56) Entgegenhaltungen:
- EP-A- 0 002 612
- EP-A- 0 551 596
- EP-A- 0 616 998
- DE-A- 19 627 679
- US-A- 4 914 231

## Beschreibung

Die vorliegende Erfindung betrifft ein Verfahren und eine Vorrichtung zur Reinigung von kristallisierbaren Verbindungen mittels gegebenenfalls fraktionierter Kristallisation, bei dem eine Schmelze oder ein Gemisch enthaltend im wesentlichen eine gewünschte Verbindung und mindestens eine Verunreinigung mindestens teilweise kristallisiert wird.

Chemische Ausgangsprodukte und Zwischenprodukte, insbesondere Ausgangsprodukte für die Polymerherstellung, werden heutzutage in sehr großen Mengen hergestellt. Um den steigenden Qualitätsanforderungen gerecht zu werden, dürfen die Produkte praktisch keine Verunreinigungen aufweisen.

In den vergangenen Jahren ist neben der Destillation zunehmend die Kristallisation als Reinigungsmethode zum Einsatz gekommen. Die letztere Methode hat den Vorteil, daß Verunreinigungen, die sich destillativ nicht entfernen lassen, häufig mittels Kristallisation abgetrennt werden können.

Die Kristallisation hat jedoch den Nachteil, daß auch gegebenenfalls in der Schmelze vorhandene Verunreinigungen beim Kristallisieren ausfallen. Wie allgemein bekannt ist, verarmt während der Kristallisation die Mutterlauge oder Schmelze am gewünschten Produkt und die Verunreinigungen reichern sich entsprechend an. Manche Verunreinigungen erreichen und überschreiten dann im Laufe des Reinigungsprozeßes ihre Löslichkeitsgrenze. Sobald die Löslichkeitsgrenze erreicht ist, fallen die Verunreinigungen aus. Diese unerwünschten Kristalle setzen sich dann am Boden oder den Wänden eines Kristallisators ab. Da die Kristalle an den Oberflächen haften, bleiben diese beim Ablassen der nicht-kristallisierten Schmelze im Kristallisator zurück. Wird dann die abgeschiedene Kristallschicht abgeschmolzen, wobei üblicherweise zwei oder mehrere Fraktionen gebildet werden, so werden die am Boden und den Wänden des Kristallisators noch vorhandenen, ausgefallenen Verunreinigungen von der erwärmten Schmelze wieder aufgenommen. Die Folge davon ist, daß die Verunreinigungen sich nie vollständig entfernen lassen und sich im Kristallisator aufkonzentrieren.

Erfolgt die Reinigung der gewünschten Verbindung wenigstens teilweise in einem statischen Kristallisator, so werden die Verunreinigungen, weil keine Bewegung der Schmelze stattfindet, teilweise auch im Kristallisat eingebaut.

Probleme der vorgenannten Art treten beispielsweise bei der Reinigung von Acrylsäure auf. Je nach Herstellungsprozeß und Effizienz der anschließenden Destillation enthält die vorgereinigte Acrylsäure einen mehr oder minder großen Anteil von Maleinsäure und von Phenothiazin (PTZ), welche Nebenprodukte in der Acrylsäureherstellung sind.

Gemäß einem in der EP-A-0 616 998 vorgeschlagenen Reinigungsverfahren erfolgt die Reinigung der Acrylsäure mittels einer Kombination von dynamischer und statischer Kristallisation, wobei der Rückstand aus der dynamischen Kristallisation mittels statischer Kristallisation gereinigt und die gereinigte Acrylsäure wieder der dynamischen Kristallisation zugeführt wird. Dies bedeutet, daß die Acrylsäureschmelze, welche dem statischen Kristallisator zugeführt wird, bereits einen hohen Anteil an Nebenprodukten wie Maleinsäure und Phenothiazin(PTZ) enthält. Wird die Schmelze dann abgekühlt, so fallen Maleinsäure und Phenothiazin (PTZ) aus und werden in der Folge auch teilweise in der abgeschiedenen Kristallschicht eingebaut.

Bei einer großtechnischen Reinigungsanlage können so im Laufe eines Tages mehr als hundert Kilo Maleinsäure anfallen. Es versteht sich von selbst, daß die feste Maleinsäure Leitungen und Ventile verstopfen kann. Auch kann sich die Maleinsäure als fester Belag an den Kristallisatorwänden oder am Boden niederschlagen. Es hat sich deshalb als eine Notwendigkeit erwiesen, die Ausfällungen abzutrennen.

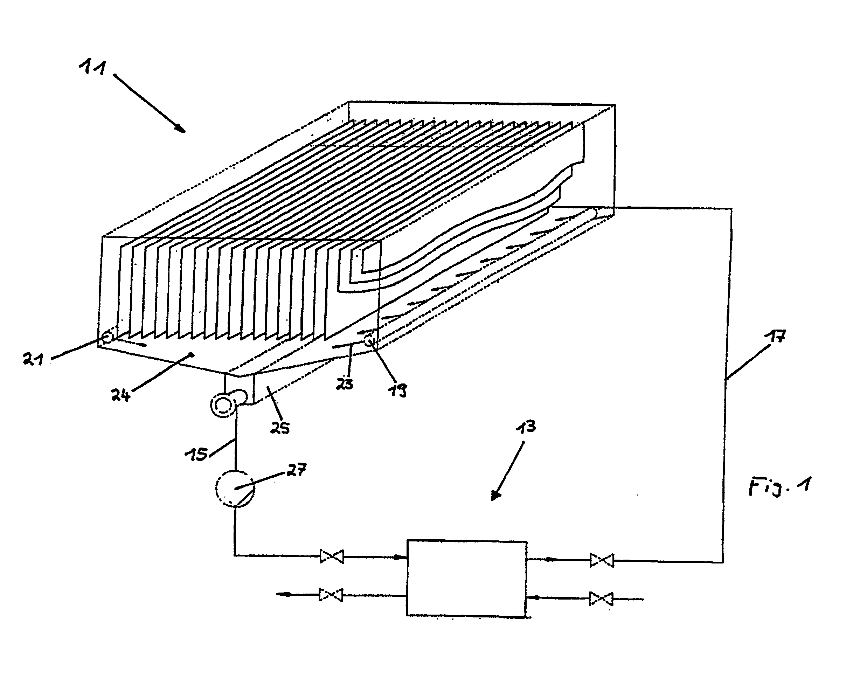

Um die ausgefällten Nebenprodukte von der Schmelze zu trennen, sind bereits verschiedene Lösungen vorgeschlagen geworden. Eine Lösung schlägt vor, die ausgefallenen Verunreinigungen auszufiltern. Wie die Figur 1 zeigt, ist ein statischer Kristallisator 11 zu diesem Zweck mit einer Abscheidevorrichtung 13 in Verbindung. Die Abscheidevorrichtung 13 ist im Normalfall ein Filter, kann jedoch auch eine Zentrifugiervorrichtung, Nutsche oder jede andere Vorrichtung sein, die die Trennung von festen und flüssigen Stoffen erlaubt. Die Abscheidevorrichtung 13 steht mittels Leitungen 15,17 mit dem Kristallisator 11 in Verbindung. Die Leitung 17 ist an Rohre 19,21 angeschlossen, welche an gegenüberliegenden Seiten im Bodenbereich des Kristallisators 11 angeordnet sind. Die Rohre 19, 21 besitzen eine Reihe von Öffnungen 23, die vorzugsweise in einem Winkel gegen den Kristallisatorboden orientiert sind. In der Mitte des im Querschnitt V-förmig ausgebildeten Bodens 24 ist ein Sammelkanal 25 vorgesehen. Dieser steht über die Leitung 15 mit der Abscheidevorrichtung 13 in Verbindung. in der Leitung 15 ist eine Pumpe 27 vorgesehen, welche dazu dient, die Schmelze umzuwälzen.

Das Abtrennen der im Laufe des Kristallisationsprozeßes ausfallenden Verunreinigungen geschieht, indem mittels der Pumpe 27 Schmelze durch die Leitung 15 abgezogen und durch die Abscheidevorrichtung 13, in welcher die Feststoffe zurückgehalten werden, gefördert wird. Die aus der Abscheidevorrichtung 13 kommende Schmelze wird dann über die Leitung 17 wieder dem Kristallisator 11 zugeführt. Vorzugsweise wird die Strömungsgeschwindigkeit der aus den Öffnungen 23 austretenden Schmelze so gewählt, daß sich eine laminare Strömung im Bereich des Kristallisatorbodens 24 einstellt. Auf diese Weise wird der Kristallisationsvorgang durch das Umwälzen der Schmelze nicht gestört. Dadurch, daß die untersten Schichten der Schmelze laufend abgezogen werden, können die sich langsam absetzenden Verunreinigen entfernt werden. Selbstverständlich kann eine solche Abscheidevorrichtung mit jeder Art Kristallisator, bei welchem das Produkt auf Kühlflächen abgeschieden wird (z.B. Fallfilm- oder statischer Kristallisator) kombiniert werden. Nicht vollständig vermeiden läßt sich mit der beschriebenen Abscheidevorrichtung allerdings der nicht erwünschte Einbau der ausfallenden Verunreinigungen in die Kristalle. Auch hat sich gezeigt, daß nicht nur Maleinsäure, sondem auch weitere Verbindungen ausfallen können.

Es ist deshalb Aufgabe der vorliegenden Erfindung ein Verfahren und eine Vorrichtung bereitzustellen, mit Hilfe derer die eingangs erwähnten Probleme weitgehend vermieden werden können. Insbesondere sollen das Verfahren und die Vorrichtung ermöglichen, Produkte, deren Reinigung mittels fraktionierter Kristallisation aufgrund von ausfallenden Verunreinigungen behindert oder unmöglich gemacht wurde, effizient zu reinigen und den Anteil der Restverunreinigungen weiter herabzusetzen. Ein weiteres Ziel ist es, ein verbessertes Verfahren und eine Vorrichtung für die Reinigung von Acrylsäure bereitzustellen.

Erfindungsgemäß geschieht dies durch ein Verfahren gemäß Anspruch 1, bei dem bei Vorhandensein von wenigstens einer Verunreinigung, die während des Reinigungsprozeßes durch Überschreiten der Löslichkeitsgrenze ausfällt, der zu reinigenden Schmelze oder dem Gemisch eine solche Menge eines Lösungsmittels oder eines Lösungsmittelgemischs zugegeben wird, daß die entsprechende Verunreinigung in Lösung gehalten wird.

Vorzugsweise besitzt das Lösungsmittel oder das Lösungsmittelgemisch zu wenigstens einer der Verunreinigungen eine hohe Affinität, d.h. das Lösungsmittel hat bevorzugt eine im Vergleich zu dem zu reinigenden Produkt verbesserte Löslichkeit für diese Verunreinigung. Dies hat den Vorteil, daß nur wenig Lösungsmittel zur Schmelze zugegeben werden muß, um ein Ausfallen der Verunreinigungen zu verhindern. Außerdem löst sich in diesem Fall nur eine geringe Menge des zu reinigenden Produkts im Lösungsmittel, so daß die Effizienz des Verfahrens erhalten bleibt.

Zweckmäßigerweise erfolgt die Wahl eines geeigneten Lösungsmittels oder eines Lösungsmittelgemisches gemäß den bekannten Löslichkeiten der Stoffe in den entsprechenden Lösungsmitteln. Wie allgemein bekannt ist, sind beispielsweise polare Verbindungen in polaren Lösungsmitteln und apolare Stoffe in apolaren Lösungsmitteln besonders gut löslich. Besonders bevorzugt wird der Schmelze ein Lösemittel zur Erhöhung der Löslichkeit von bestimmten Verunreinigungen zugesetzt, welches bereits im Einsatzprodukt als Verunreinigung vorhanden ist.

Die erfindungsgemäße Weiterentwicklung der bekannten Kristallisationsverfahren kann bei der Reinigung beliebiger Stoffe angewendet werden, in deren Verlauf es zu Ausfällungen von Verunreinigungen kommt. Vorzugsweise wird als Kristallisationsverfahren die fraktionierte Kristallisation eingesetzt. Mittels fraktionierter Kristallisation zu reinigende Substanzen sind insbesondere Acrylsäure, Methacrylsäure, Abwasser, Methylendiphenylisocyanat (MDI), Toluoldiisozyanat (TDI), Caprolactam, Benzoesäure, Bisphenol-A-, Nitrochlorbenzol, geradkettige und verzweigte Fettsäuren, Hydrazin, Phenole wie para-, meta und ortho-Kresol, 2.6 und 3,5-Dimethylphenol, Naphthol und o,o-Diphenol, chlorierte Kohlenwasserstoffe wie Dichlorbenzol und Naphthalin, 1- 2-Methylnaphthalin, Acenaphthen, Fluoren, Phenanthren, Adipinsäuredinitril, Hexamethylendiamin, sowie Paraffine ab C₁₇.

Insbesondere eignet sich das erfindungsgemäße Verfahren zur Reinigung von Acrylsäure durch Abtrennung von Maleinsäure und/oder deren Anhydrid und gegebenenfalls weiteren Verunreinigungen.

Gegenstand der vorliegenden Erfindung ist auch eine Kristallisationsvorrichtung und -anlage, welche sich von bekannten Vorrichtungen dadurch unterscheidet, daß an der Vorrichtung eine Anschlußstelle zum Zudosieren eines Lösungsmittels oder eines Lösungsmittelgemisches vorgesehen ist. Das Lösungsmittel wird vorzugsweise in einen Vorratstank oder einen Tank zum Zwischenlagern der anfallenden Fraktionen zugegeben. Ebenfalls bevorzugt ist auch die Zugabe des Lösungsmittel direkt in den Kristallisator.

Nachfolgend werden Ausführungsbeispiele der Erfindung unter Bezugnahme auf die Figuren beschrieben. Die Ausführungen sind lediglich beispielhaft und schränken somit den allgemeinen Erfindungsgedanken nicht ein. Es zeigen:
- **Figur 1**: Schematisch einen statischen Kristallisator mit einer Abscheidevorrichtung;
- **Figur 2**: Schematisch einen erfindungsgemäßen Kristallisator
- **Figur 3**: Ebenfalls schematisch eine erfindungsgemäße Kristallisationsanlage.

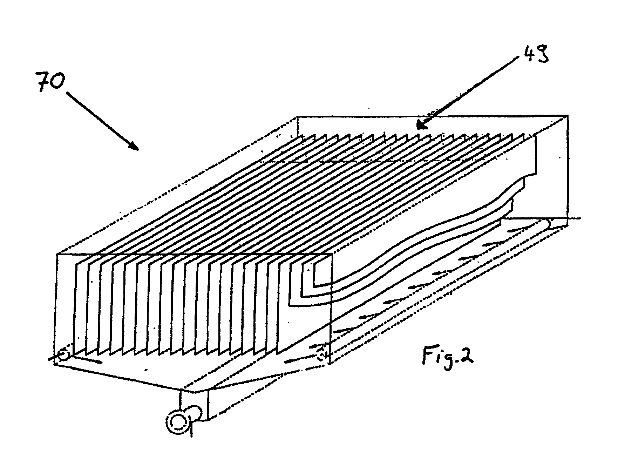

**Figur 2** zeigt den erfindungsgemäßen Kristallisator 70. Bei diesem Kristallisator 70 handelt es sich um den Kristallisator gemäß Figur 1, der jedoch zusätzlich eine Anschlußstelle 49 aufweist, mit der ein Lösungsmittel in den Kristallisator dosiert werden kann. Der Fachmann erkennt, daß der Kristallisator jeder beliebige Kristallisator sein kann.

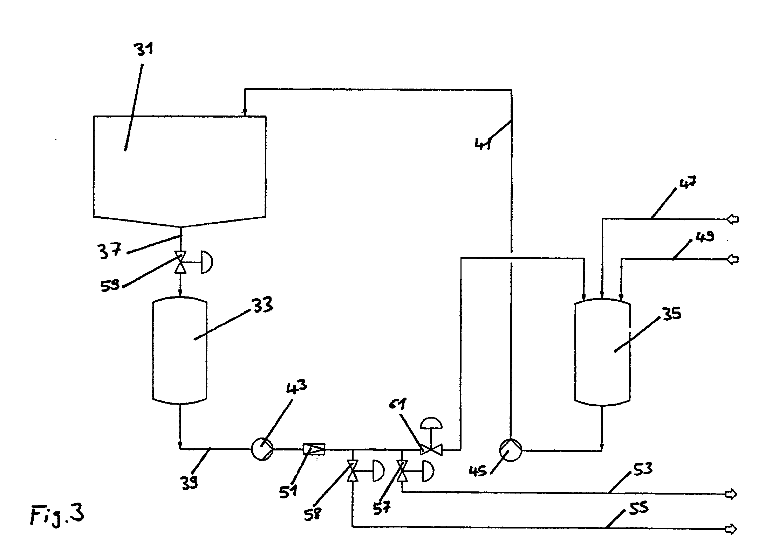

Die in **Figur 3** schematisch dargestellte Kristallisationsanlage besteht im wesentlichen aus einem nicht näher spezifizierten Kristallisator 31 eines bekannten Typs, einem Meßtank 33, welcher dem Kristallisator 31 nachgeschaltet ist, und einem sogenannten Stufentank 35, in welchem das Einsatzmaterial zwischengelagert wird. Kristallisator 31, Meßtank 33 und Stufentank 35 stehen über Leitungen 37,39,41 miteinander in Verbindung. Förderpumpen 43,45 dienen dem Transport der Schmelze vom Meßtank 33 in den Stufentank 35, respektive vom letzteren in den Kristallisator 31.

Das Besondere an der Kristallisationsanlage ist, daß der Tank 35 neben einer Anschlußtelle 47 für die Zuführung von Einsatzmaterial eine weitere Anschlußtelle 49 für die Zuführung eines Lösungsmittels oder eines Lösungsmittelgemisches vorgesehen ist. Eine nicht näher dargestellte Dosiervorrichtung erlaubt es, eine dem Verunreinigungsanteil entsprechende Lösungsmittelmenge zuzudosieren, so daß ein Ausfallen der Verunreinigungen verhindert werden kann.

Dennoch auftretende Ausfällungen können mit Hilfe eines Filters 51, welcher in der Leitung 39 vorgesehen ist, herausfiltriert werden.

Der Fachmann erkennt, daß die Dosierung des Lösungsmittel automatisch erfolgen kann. Sobald sich z.B. Kristalle einer bestimmten Zusammensetzung in dem Filter 51 ansammeln, wird die Dosierung des Lösungsmittels erhöht.

Zwei separate Leitungen 53,55, welche an die Leitung 39 angeschlossen sind, ermöglichen via der Absperrventile 57,58 die Entnahme des gereinigten Produkts oder des übrigbleibenden Rückstandes. Weitere Absperrventile 59,61 sind zwischen dem Kristallisator 31 und dem Meßtank 33, bzw. dem Meßtank 33 und dem Tank 35 vorgesehen.

Es versteht sich von selbst, daß die in **Figur 2** nur schematisch dargestellte Anlage in Wirklichkeit mehrere Tanks und Kristallisatoren aufweisen kann. Beispielhafte Anlagen sind in der EP-A-0 616 998 offenbart.

Das erfindungsgemäße Verfahren soll nachfolgend beispielhaft anhand der Acrylsäurereinigung näher erläutert werden.

Durch Destillation vorgereinigte Acrylsäure kann neben anderen Verunreinigungen Maleinsäure, deren Anhydrid und Phenotiazin enthalten, die bei Konzentrationen von über 4% (Maleinsäure) bzw. 1.5% (Phenotiazin) aus der Schmelze ausfallen. Aus den nachfolgenden Beispielen (Tabelle 1 und 2) ist ersichtlich, wieviel Feststoff jeweils aufgrund des Überschreitens der Löslichkeitsgrenze während eines Kristallisationsvorgangs ausfällt.

In den Tabellen 1, 2 und 3 zeigt die erste Zeile jeweils die Zusammensetzung des Einsatzmaterials, die zweite Zeile die Zusammensetzung der (durch statische Kristallisation) gereinigten Acrylsäure, welche dann durch Fallfilm-Kristallisation weiter gereinigt werden kann, und die dritte Zeile die Zusammensetzung des anfallenden Rückstandes.

Wie aus der Tabelle 2 ersichtlich ist, fallen 17.3 Kg Rückstand (= Mutterlauge) an, von denen 3.6% (= 0.62 Kg) PTZ sind. Da die Löslichkeit von PTZ jedoch lediglich ca. 1.5% beträgt, fallen 0.4 Kg PTZ als Feststoff an.

Aus Tabelle 1 wird ersichtlich, daß es sich im Fall von Maleinsäure und deren Anhydrid ähnlich verhält, wobei die in Tabelle 1 angegebenen Zahlenwerte Maleinsäure und deren Anhydrid angegeben als Maleinsäure beinhalten. Die Löslichkeit von Maleinsäure in Acrylsäure beträgt ca. 4%. Der Rückstand (13.2 Kg) enthält 20.4% oder 2.7 Kg Maleinsäure, von welcher wegen Überschreitens der Löslichkeitsgrenze 2.4 Kg als Feststoff ausfallen.

Werden dem Einsatzmaterial, welches insgesamt 15% Verunreinigungen enthält (vgl. Tabelle 1 und 2), ein für Maleinsäure und deren Anhydrid gut lösliches Lösungsmittel zugegeben, so kann die Maleinsäure vollständig in Lösung gehalten werden, d.h. es kommt zu keinen Ausfällungen mehr.

Da die Maleinsäure und deren Anhydrid bekanntlich in Wasser sehr gut löslich ist, kann durch Zugabe Wasser zur Acrylsäure-Schmelze ein Ausfallen der Maleinsäure verhindert werden, auch wenn sich diese während des Kristallisationsvorgangs in der Mutterlauge weiter anreichert. Im gezeigten Beispiel (Tabelle 3) ist eine Zugabe von 6.8 Kg Wasser nötig, um die Maleinsäure im Rückstand in Lösung zu halten. Die Zugabe von Wasser ist insofern vorteilhaft, als Wasser in der Roh-Acrylsäure bereits als Verunreinigung vorkommt, d.h. es wird durch die Wasserzugabe keine weitere Verunreinigung eingebracht.

Da sich Phenotiazin in Wasser nur in geringen Mengen löst (unterschiedliche Polaritäten), wird die Löslichkeit von Phenotiazin durch die Zugabe von Wasser nicht wesentlich beeinflußt, d.h. es kommt bei gleichzeitigem Vorhandensein von einem Anteil von mehr als 1.5% Phenotiazin in der Mutterlauge weiterhin zu einer Ausfällung von Phenotiazin.

Gemäß einer vorteilhaften ersten Ausgestaltung des erfindungsgemäßen Verfahrens wird deshalb vorgeschlagen, eine Mischung von Wasser mit einem niedermolekularen Alkohol (Methanol, Ethanol, n- oder i-Propanot) zuzusetzen, um sowohl die Maleinsäure als auch das Phenotiazin in Lösung zu halten. Grundsätzlich kann auch jedes andere Lösungsmittelgemisch zum Einsatz kommen, mit welchem es gelingt, die Löslichkeitsgrenze für beide Stoffe, die zur Ausfällung neigen, heraufzusetzen.

Da durch die Zugabe von Lösungmittels die Schmelze weiter abgekühlt werden muß, führt dies zu einer Verschlechterung der Energiebilanz des Prozeßes. Es ist daher ein Bestreben, die Zugabe von Lösungsmittel möglichst gering zu halten.

Alternativ wird deshalb ein kombiniertes Verfahren vorgeschlagen, nämlich, das Phenotiazin, welches im Vergleich zu Maleinsäure in wesentlich geringeren Mengen anfällt, abzutrennen, und die Maleinsäure und deren Anhydrid durch die Zugabe von Wasser in Lösung zu halten. Durch die Kombination der beiden Verfahren läßt sich der Prozeß unter Einbezug der Energiebilanz optimal durchführen.

### Bezugsziffern

- 11: stat. Kristallisator
- 13: Abscheidevorrichtung
- 15,17: Leitungen
- 19,21: Rohre
- 23: Öffnungen der Rohre
- 25: Sammelkanal
- 31: Kristallisator
- 33: Meßtank
- 35: Stufentank
- 37,39,41: Leitungen
- 43,45: Förderpumpen
- 47: Anschlußtelle am Tank für Einsatzmaterial
- 49: Anschlußtelle am Tank für Lösungsmittel
- 51: Filter
- 53,55: Leitungen
- 57,58: Absperrventile der Leitungen 53,55
- 59,61: Absperrventile
- 70: Erfindungsgemäßer Kristallisator

**Tabelle 1:**

| Menge [in Kg] | Gewicht | Maleinsr. [in %] | Acrylsr. [in %] | andere Verunreinigungen [in %] |
|---|---|---|---|---|
| Einsatzmaterial | 100 | 4.0 | 85.0 | 11.0 |
| gereinigte Fraktion | 86.8 | 1.5 | 88.4 | 10.1 |
| Rückstand | 13.2 | 20.4 | 62.7 | 16.8 |

| Anreicherung von Maleinsäure im Rückstand bei der Acrylsäurereinigung (ohne Zugabe von Lösungsmittel) | | | | |
|---|---|---|---|---|
| | | | | |

**Tabelle 2:**

| Menge [in Kg] | Gewicht | Phenotiazin [in %] | Acrylsr. [in %] | andere Verunreinigungen [in %] |
|---|---|---|---|---|
| Einsatzmaterial | 100 | 1.4 | 85.0 | 13.6 |
| Gereinigte Fraktion | 82.7 | 0.9 | 89.6 | 9.4 |
| Rückstand | 17.3 | 3.6 | 62.8 | 33.6 |

| Anreicherung von Phenotiazin im Rückstand bei der Acrylsäurereinigung (ohne Zugabe von Lösungsmittel) | | | | |
|---|---|---|---|---|
| | | | | |

**Tabelle 3:**

| Menge [in Kg] | Gewicht | Maleinsr. [in %] | Acrylsr. [in %] | Andere Verunreinigungen [in %] | Lösungsmittel [in %] |
|---|---|---|---|---|---|
| Einsatzmaterial | 106.8 | 3.7 | 79.6 | 10.3 | 6.4 |
| Gereinigte Fraktion | 87.6 | 1.3 | 86.8 | 9.8 | 2.1 |
| Rückstand | 19.2 | 15.1 | 46.5 | 12.5 | 25.9 |

| Anreicherung von gelöster Maleinsäure im Rückstand bei der Acrylsäurereinigung mit Zugabe von Lösungsmittel) | | | | | |
|---|---|---|---|---|---|
| | | | | | |

## Patentansprüche

1. Verfahren zur Reinigung von kristallisierbaren Verbindungen mittels gegebenenfalls fraktionierter Kristallisation, bei dem eine Schmelze oder ein Gemisch enthaltend im wesentlichen die gewünschte Verbindung und mindestens eine Verunreinigung mindestens teilweise kristallisiert werden, **dadurch gekennzeichnet, dass** der Schmelze oder dem Gemisch eine solche Menge eines Lösungsmittels oder eines Lösungsmittelgemisches zugegeben wird, daß mindestens eine zum Ausfallen neigende Verunreinigung in Lösung gehalten wird, und daß bei Vorhandensein von zwei oder mehreren zum Ausfallen neigenden Verunreinigungen wenigstens die quantitativ bedeutendste Verunreinigung durch Zugabe eines geeigneten Lösungsmittels oder eines Lösungsmittelgemisches in Lösung gehalten wird und daß die restlichen Verunreinigungen, die trotz Zugabe des Lösungsmittels oder des Lösungsmittelgemisches immer noch ausfallen, durch eine Abscheidevorrichtung (51) von der Schmelze abgetrennt werden.

2. Verfahren nach Anspruch 1, **dadurch gekennzeichnet, daß** das Lösungsmittel im Vergleich zu dem zu reinigenden Produkt eine verbesserte Löslichkeit für wenigstens eine der zum Ausfallen neigenden Verunreinigungen aufweist.

3. Verfahren nach einem der Ansprüche 1 oder 2, **dadurch gekennzeichnet, daß** bei Vorhandensein von zwei oder mehreren zum Ausfallen neigenden Verunreinigungen ein Lösungsmittel oder ein Lösungsmittelgemisch zugesetzt wird, welches geeignet ist, mehrere Verunreinigungen in Lösung zu halten.

4. Verfahren nach Anspruch 1 bis 3, **dadurch gekennzeichnet, daß** das gebildete Kristallisat, welches die gewünschte Verbindung in gereinigter Form enthält, aufgeschmolzen und die Schmelze gegebenenfalls in Fraktionen gesammelt wird, wobei die anfallenden Fraktionen bei Bedarf weiteren Kristallisations/Schmelzzyklen unterworfen werden.

5. Verfahren zur Reinigung von verunreinigter Acrylsäure mittels Kristallisation, **dadurch gekennzeichnet, daß** einer Schmelze oder einem Gemisch enthaltend Acrylsäure und Verunreinigungen ein Wasser/Alkohol-Gemisch zugesetzt wird.

6. Verfahren nach Anspruch 5, **dadurch gekennzeichnet, daß** von der Acrylsäure Maleinsäure deren Anhydrid und/oder Phenothiazin und gegebenenfalls weitere Verunreinigungen abgetrennt werden.

7. Verfahren nach Anspruch 5 bis 6, **dadurch gekennzeichnet, daß** andere Verunreinigungen, die ausfallen durch mindestens eine Abscheidevorrichtung (51) abgetrennt werden.

8. Verfahren nach Anspruch 7, **dadurch gekennzeichnet, daß** die Abscheidevorrichtung ein Filter, eine Nutsche oder eine Zentrifuge ist.

9. Kristallisator (70), **dadurch gekennzeichnet, daß** er eine Anschlußstelle (49) zum Dosieren eines Lösungsmittels oder eines Lösungsmittelgemisches aufweist, die mit einer Dosiervorrichtung für ein lösungsmittel oder ein lösungsmittelgemisch in verbindung steht.

10. Kristallisationsanlage mit wenigstens einem Kristallisator (31) und wenigstens einem Tank (35) zur Aufnahme einer zu reinigenden Verbindung oder eines zu reinigenden Stoffgemisches, Leitungen (37,39,41) und Anschlußstelle zum Zuund Abführen der Verbindung in den Kristallisator (31) und in den Tank (35), sowie mit den Leitungen (37,39,41) in Verbindung stehenden Förderpumpen (43,45) zum Fördern der Verbindung in den Leitungen (37,39,41), **dadurch gekennzeichnet, daß** sie eine Anschlußstelle (49) zum Dosieren eines Lösungsmittels oder eines Lösungsmittelgemisches ausweist, die mit einer Dosiervorrichtung für ein lösungsmittel oder ein lösungsmittelgemisch in verbindung steht.

11. Kristallisationsanlage nach Anspruch 10, **dadurch gekennzeichnet, daß** die Anschlußstelle (49) an dem Tank (35) angeordnet ist

12. Kristallisationsanlage nach Anspruch 10 oder 11, **dadurch gekennzeichnet, daß** sie wenigstens eine mit dem Kristallisator in Verbindung stehende Abscheidevorrichtung (51) aufweist, um Ausfällungen von der Schmelze abzutrennen.

13. Kristallisationsanlage nach Anspruch 12, **dadurch gekennzeichnet, daß** die Abscheidevorrichtung ein Filter, eine Nutsche und/oder eine Zentrifuge ist

## Claims

1. A process for the purification of crystallizable compounds by means of optionally fractional crystallization, wherein a molten material or a mixture containing essentially the desired compound and at least one impurity is subjected to at least partial crystallization, **characterized in that** the molten material or the mixture is added with such an amount of a solvent or a mixture of solvents that at least one impurity having a tendency to precipitate is retained in solution, and that in case two or more impurities having a tendency to precipitate are present, at least the quantitatively more significant impurity is retained in solution by adding a suitable solvent or a mixture of solvents, and that the residual impurities still precipitating despite addition of said solvent or mixture of solvents are removed from the molten material by means of a separator (51).

2. The process according to claim 1, **characterized in that** the. solvent has a superior dissolving capacity for at least one of the impurities having a tendency to precipitate, compared to the product to be purified.

3. The process according to one of claims 1 or 2, **characterized in that** in case two or more impurities having a tendency to precipitate are present, a solvent or a mixture of solvents is added which is suitable in retaining multiple impurities in solution.

4. The process according to claims 1 to 3, **characterized in that** the crystallized product formed, which contains the desired compound in a purified form, is melted and the molten material is collected, optionally in fractions, the fractions obtained being subjected to further crystallization/melt cycles, if necessary.

5. A process for the purification of contaminated acrylic acid using crystallization, **characterized in that** a molten material or a mixture containing acrylic acid and impurities is added with a water/alcohol mixture.

6. The process according to claim 5, **characterized in that** maleic acid, its anhydride and/or phenothiazine and optionally other impurities are separated from the acrylic acid.

7. The process according to claims 5 to 6, **characterized in that** other precipitating impurities are removed by at least one separator (51).

8. The process according to claim 7, **characterized in that** the separator is a filter, a suction filter or a centrifuge.

9. A crystallizer (70), **characterized in that** it includes a connecting piece (49) for metering a solvent or a mixture of solvents, which is in connection with a metering device for a solvent or a mixture of solvents.

10. A crystallization plant having at least one crystallizer (31) and at least one tank (35) for receiving a compound to be purified or a mixture of substances to be purified, pipes (37, 39, 41) and a connecting piece for supplying and discharging the compound to/from the crystallizer (31) and to/from the tank (35), as well as conveying pumps (43, 45) connected with pipes (37, 39, 41) to convey the compound through said pipes (37, 39, 41), **characterized in that** it includes a connecting piece (49) for metering a solvent or a mixture of solvents, which is in connection with a metering device for a solvent or a mixture of solvents.

11. The crystallization plant according to claim 10, **characterized in that** the connecting piece (49) is arranged on tank (35).

12. The crystallization plant according to claim 10 or 11, **characterized in that** it includes at least one separator (51) connected with the crystallizer in order to remove precipitations from the molten material.

13. The crystallization plant according to claim 12, **characterized in that** the separator is a filter, a suction filter and/or a centrifuge.

## Revendications

1. Procédé de purification de composés pouvant être cristallisés au moyen de cristallisation, fractionnée, le cas échéant dans le cas duquel une matière fondue ou un mélange comportant essentiellement le composé désiré et au moins une impureté sont cristallisés au moins partiellement, **caractérisé en ce qu'**on ajoute à la matière fondue ou au mélange une telle quantité d'un solvant ou d'un mélange de solvant qu'au moins une impureté ayant la tendance à précipiter est maintenue en solution et **en ce qu'**en présence de deux ou plusieurs impuretés ayant la tendance à précipiter au moins l'impureté la plus importante quant à sa quantité est maintenue en solution par l'addition d'un solvant approprié ou d'un mélange de solvant et **en ce que** les impuretés restantes qui sont encore précipités malgré l'addition du solvant ou du mélange de solvant sont séparées de la matière fondue au moyen d'un dispositif séparateur (51).

2. Procédé selon la revendication 1, **caractérisé en ce qu'**en comparaison avec le produit devant être purifié le solvant présente une solubilité améliorée pour au moins une des impuretés ayant la tendance à précipiter.

3. Procédé selon l'une des revendications 1 ou 2, **caractérisé en ce qu'**en présence de deux ou de plusieurs impuretés ayant la tendance à précipiter un solvant ou un mélange de solvant est ajouté qui est approprié à maintenir en solution plusieurs impuretés.

4. Procédé selon la revendication 1 à 3, **caractérisé en ce que** le produit cristallisé qui comporte le composé désiré sous forme purifiée est fondu et la matière fondue est recueillie, le cas échéant en fractions, les fractions obtenues étant soumises à d'autres cycles de cristallisation et de fusion, si nécessaire.

5. Procédé de purification d'acide acrylique impur au moyen de cristallisation, **caractérisé en ce qu'**on ajoute un mélange d'eau/alcool à une matière fondue ou à un mélange comportant de l'acide acrylique et des impuretés.

6. Procédé selon la revendication 5, **caractérisé en ce que** de l'acide maléique, son anhydride et/ou de la phénothiazine et le cas échéant d'autres impuretés sont séparées de l'acide acrylique.

7. Procédé selon la revendication 5 à 6, **caractérisé en ce que** d'autres impuretés qui sont précipitées sont séparées par au moins un dispositif séparateur (51).

8. Procédé selon la revendication 7, **caractérisé en ce que** le dispositif séparateur est un filtre, un filtre de succion ou une centrifugeuse.

9. Cristallisateur (70), **caractérisé en ce qu'**il présente un endroit de raccord (49) pour le dosage d'un solvant ou d'un mélange de solvant qui est en liaison avec un dispositif de dosage pour un solvant ou un mélange de solvant.

10. Installation de cristallisation avec au moins un cristallisateur (31) et au moins un réservoir (35) pour recevoir un composé devant être purifié ou un mélange de substances devant être purifié, des conduites (37, 39, 41) et un endroit de raccord pour alimenter et pour décharger le composé dans le cristallisateur (31) et dans le réservoir (35) ainsi qu'avec des pompes d'alimentation (43, 45) étant en liaison avec les conduites (37, 39, 41) pour transporter le composé dans les conduites (37, 39, 41), **caractérisée en ce qu'**elle a un endroit de raccord (49) pour le dosage d'un solvant ou d'un mélange de solvant qui est en liaison avec un dispositif de dosage pour un solvant ou un mélange de solvant.

11. Installation de cristallisation selon la revendication 10, **caractérisée en ce que** l'endroit de raccord (49) est agencé sur le réservoir (35).

12. Installation de cristallisation selon la revendication 10 ou 11, **caractérisée en ce qu'**elle présente au moins un dispositif séparateur (51) étant en liaison avec le cristallisateur pour séparer des précipités de la matière fondue.

13. Installation de cristallisation selon la revendication 12, **caractérisée en ce que** le dispositif séparateur est un filtre, un filtre de succion et/ou une centrifugeuse.
